# EUROPÄISCHE PATENTSCHRIFT

(11) **EP 1 099 430 B1**
(45) Veröffentlichungstag und Bekanntmachung des Hinweises auf die Patenterteilung: **04.02.2004**
(21) Anmeldenummer: 99122314.0
(22) Anmeldetag: 09.11.1999
(51) Int. Cl.: A61F 2/46, A61B 17/15, A61F 2/38

(54) **Knieprothesensystem**
Knee prosthesis system
Système pour une prothèse de genou

(43) Veröffentlichungstag der Anmeldung: 16.05.2001
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 824 904
- WO-A-79/00739
- FR-A- 2 589 720
- US-A- 3 949 428

## Beschreibung

Bekanntlich enthält das Kniegelenk zwei tibio-femorale Gelenkflächenpaare, nämlich ein mediales und ein laterales Gelenkflächenpaar, die jeweils von einem femoralen Kondylus und einer damit zusammenwirkenden, schalenförmigen tibialen Gelenkfläche gebildet sind. Ungekoppelte Knieprothesen bilden die natürlichen Verhältnisse insofern nach, als sie gleichfalls zwei Gelenkflächenpaare aufweisen. Die beiden tibiaseitig bzw. femoral vorgesehenen Gelenkflächen werden dabei in neuerer Zeit in der Regel zu einem Stück zusammengefaßt (US-A 4 081 866; WO 79/00739, Fig. 39-41), weil sich dadurch eine genauere Ausrichtung der Gelenkflächen zueinander und zum Knochen erzielen läßt. Ausnahmsweise werden die Kondylengleitflächen oder die tibialen Gleitflächen auch noch je einzeln ersetzt (DE-U 6949817; WO 79/00739, Fig. 34, 58-60). Zur leichteren Ausrichtung der Prothesenteile verwendet man in diesem Fall Einsetzinstrumente, die entsprechend aufeinander ausgerichtete Halteeinrichtungen für die Prothesenteile bilden. Ein bekanntes Einsetzinstrument (W. LINK: Prospekt "Schalen-Kniegelenkprothesen-System SKI") dient zum Einsetzen von Tibiaplateaus, deren gegenseitiger Abstand stufenlos verstellbar ist, damit er den jeweiligen anatomischen Verhältnissen angepaßt werden kann. Da ihre Gleitflächen sehr flach oder eben sind, kommt es auf ihren Abstand im Verhältnis zu den in diesem Fall einstückig verbundenen femoralen Gleitflächen nicht an. Ein anderes bekanntes Einsetzinstrument (EP-A 824 904) ist für femorale Gleitflächen bestimmt und gestattet keine Änderung des gegenseitigen Abstands der Prothesenteile. Ein weiteres bekanntes Einsetzinstrument (US-A 3,949,428) wird sowohl für die femoralen als auch für die tibialen Prothesenteile verwendet. Der Abstand der für diese Teile an dem Instrument vorgesehenen Halterungen ist verstellbar, wobei zunächst die tibialen und femoralen Prothesenteile mit derselben Abstandseinstellung eingesetzt werden.

Die oben erwähnte, einstückige Ausführung der femoralen Gelenkflächenteile hat sich auch deshalb in neuerer Zeit durchgesetzt, weil dies die Möglichkeit bietet, eine Gleitfläche für die Patella oder einen Patellaersatz zu bilden, der als Patellaschild die Gleitflächenteile verbindet (US-A 4,081,866). Da derartige Prothesen sich bewährt haben, verwendet man sie häufig auch dann, wenn die femorale Patellagleitbahn noch gut erhalten ist und eigentlich nicht ersetzt zu werden brauchte. Zwar gibt es Kniegelenkprothesen ohne Patellaschild (US-A 3,728,742), bei denen die Gelenkflächenteile lediglich durch einen verhältnismäßig dünnen Steg miteinander verbunden sind, der ihre Ausrichtung bei der Implantation erleichtern soll. Jedoch hat sich gezeigt, daß dieser Steg verhältnismäßig häufig bricht und dann zu Störungen führen kann.

Die Erfindung setzt ein Knieprothesensystem voraus, das ein einstückiges Tibiaplateau mit zwei Gleitmulden und eine einstückige Femurkomponente mit zwei Kondylengleitflächen umfaßt, deren Abstand und Form auf die tibialen Gleitmulden abgestimmt ist. Sie schafft zusätzlich eine Implantationsalternative für diejenigen Fälle, in denen die femorale Patellagleitfläche noch so gut erhalten ist, daß sie nicht ersetzt zu werden braucht, indem sie diesem System als Alternative zu der einstückigen Femurkomponente ein Paar von unverbundenen, femoralen Kondylenschalen hinzufügt, deren Gleitflächenform ebenso auf die tibialen Gleitmulden abgestimmt ist wie die Kondylengleitflächen der einstückigen Femurkomponente. Damit sie auch relativ zueinander genau diejenige Position einnehmen, die die Kondylen der einstückigen Komponente zueinander aufweisen, gehört zu dem System außerdem ein Einsetzinstrument für die Kondylenschalen, das ihnen eine auf die tibialen Gleitmulden abgestimmte relative Position verleiht.

Die Erfindung ermöglicht es, ein und dasselbe Knieprothesensystem für unterschiedliche Schadenszustände zu verwenden. Wenn die gesamten Gleitflächen des Ersatzes bedürfen, wird die einstückige Kondylenkomponente verwendet. Wenn hingegen die Patellagleitflächen sich noch in gutem Zustand befinden, werden lediglich die Kondylengleitflächen ersetzt. Zudem ist es möglich, durch einen vergleichsweise kleineren Eingriff die Kondylenschalen durch eine einstückige Femurkomponente zu ersetzen, wenn sich im Laufe der Zeit herausstellen sollte, daß auch die Patellagleitflächen des Ersatzes bedürfen. Schließlich ist auch ein intraoperativer Wechsel von der zunächst vorgesehenen, weniger weitgehenden Variante zur weitergehenden Variante möglich.

Dabei ist es nicht erforderlich, auf komplizierte Gleitflächenformen, wie sie bei einstückigen Prothesen sich entwikkelt haben, zu verzichten. Bislang ist es nämlich üblich, getrennte Prothesenschalen und/oder die zugehörigen Tibiagleitflächen so zu gestalten, daß sie Implantationsungenauigkeiten verzeihen, weil ihre Formen nicht genau aufeinander abgestimmt zu werden brauchen. Jedoch ist es im Rahmen des erfindungsgemäßen Systems durch das Hinzukommen des Einsetzinstruments möglich, die getrennten Prothesenschalen ebenso abgestimmt auf die Tibiakomponente zu formen, wie die entsprechenden Kondylenteile der einstückigen Femurkomponente, weil Gewähr für eine hinreichend genaue Implantation besteht. Form und Abstand der Gleitflächen der Kondylenschalen können daher mit der Form und dem Abstand der Kondylengleitflächen der einstückigen Femurkomponente übereinstimmen.

Ferner ist es zweckmäßig, wenn die kondylären Resektionsflächen, die für das Einsetzen der Kondylenschalen erforderlich sind, mit den entsprechenden Resektionsflächen der einstückigen Femurkomponente übereinstimmen. Das hat nicht nur den Vorteil, daß dieselbe Sägelehre verwendet werden kann. Vielmehr wird dadurch auch der Übergang von einer femoralen Prothesenvariante zur anderen erleichtert.

Da die einstückige Femurkomponente sich über die gesamte Breite der Femurkondylen erstreckt, sind die zugehörigen Sägeschnitte nicht nur im Bereich der Kondylen, sondern auch im Zwischenbereich zu führen. Letzteres ist bei Verwendung von Kondylenschalen nicht erforderlich. Es kann sogar schädlich sein, wenn - je nach Lage der Resektionsschnitte - dadurch auch ein Teil des femoralen Gleitflächenbereichs der Kniescheibe erfaßt werden könnte. Erfindungsgemäß ist deshalb vorgesehen, daß die Sägelehre, die für beide Varianten von Femurprothesen verwendet wird, im Zwischenkondylenbereich einen Sperransatz aufweist, der es dem Operateur gestattet, den Zwischenkondylenbereich zu schonen oder sogar die Kondylenschnitte an den einander zugewendeten Seiten genau entsprechend der vorgesehenen Lage der Kanten der Kondylenschalen zu begrenzen.

Die Begrenzungen der Kondylenschalen können in beliebiger Form gewählt sein. Jedoch ist es zweckmäßig, wenn sie einander parallele, gerade Seitenflächen zukehren. Dann fällt nämlich ihre genaue Ausrichtung am Einsatzinstrument leichter, wenn dies dazu passende parallele Anschlußflächen aufweist.

Das erfindungsgemäße Prothesensystem enthält normalerweise mehrere Größenstufen mit unterschiedlichen Abständen der Kondylenschalen. Entsprechend sieht die Erfindung vor, daß der gegenseitige Abstand der parallelen Anschlußflächen des Einsatzinstruments stufenweise unterschiedlich einstellbar ist. Ihre Verstellbarkeit beruht zweckmäßigerweise auf der Verwendung von Wechselteilen, die jeweils nur einer Stufe zugeordnet sind, weil dadurch die Gefahr einer fehlerhaften Einstellung des Abstands der Kondylenschalen verringert wird. Eine bevorzugte Ausführungsform der Erfindung sieht vor, daß die Anschlußflächen des Einsatzinstruments - seien sie nun parallel oder nicht - von je einer feststehenden Backe einzelner, jeweils einer Kondylenschale zugeordneter Klemmen gebildet sind, für die nach einem weiteren Merkmal unterschiedliche, den einzelnen Größenstufen zugeordnete Halterungen verwendet werden.

Weitere vorteilhafte Merkmale der Erfindung ergeben sich aus der folgenden Beschreibung eines Ausführungsbeispiels anhand der Zeichnungen. Darin zeigen:
- Fig. 1: eine Gesamtansicht der Prothese mit einstückiger femoraler Komponente,
- Fig. 2: dasselbe mit zweistückiger femoraler Komponente,
- Fig. 3: die Sägelehre,
- Fig. 4: das Einsetzinstrument,
- Fig. 5: das Einsetzinstrument mit daran angebrachten Kondylenschalen in Draufsicht,
- Fig. 6: eine perspektivische Teilansicht des Einsetzinstruments,
- Fig. 7: das Einsetzinstrument mit Kondylenschalen beim Aufsetzen derselben auf den Knochen,
- Fig. 8: die am Knochen fixierten Probeschalen nach dem Abnehmen des Einsetzinstruments und
- Fig. 9: die Prothesenschalen im angesetzten Zustand.

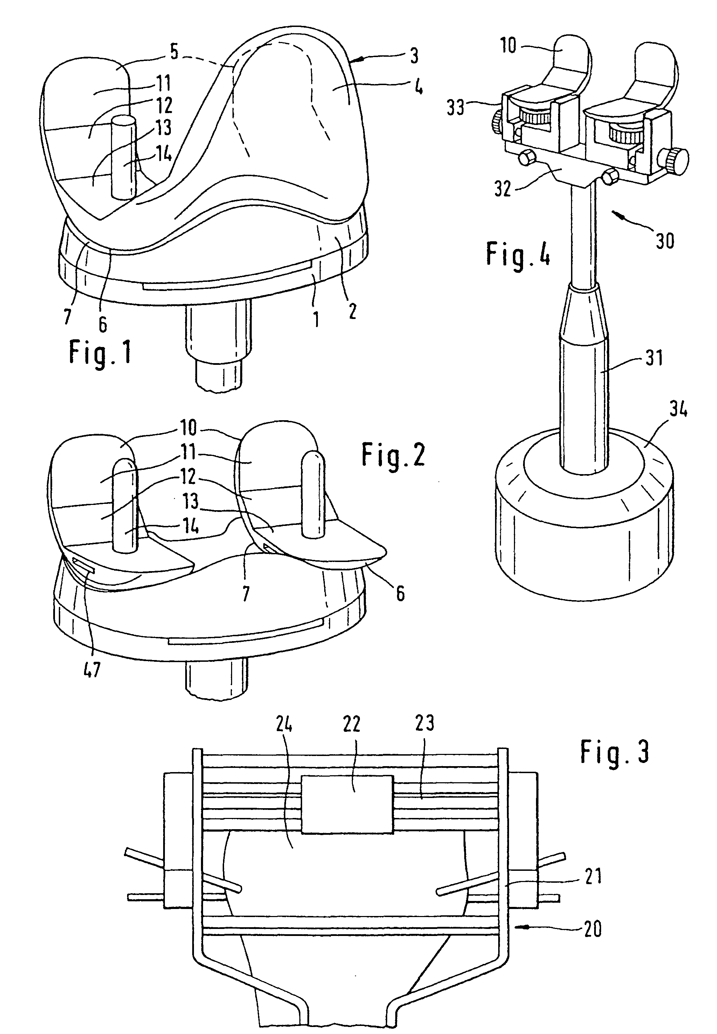

Gemäß Fig. 1 besteht die Prothese aus einer am oberen Ende des Schienbeins zu verankernden Tibiaplatte 1, einem darauf drehbar gehaltenen Tibiaplateau 2 aus Polyethylen und einer einstückigen Femurkomponente 3, die ein Patellaschild 4 zur Bildung einer Patellagleitfläche sowie Kondylenteile 5 zur Bildung von Kondylengleitflächen 6 aufweist. Das Tibiaplateau 2 enthält zur Aufnahme der Kondylengleitflächen 6 entsprechend geformte Mulden 7. Infolge der einstückigen Ausbildung des Tibiaplateaus 2 der Femurkomponente 3 ist Gewähr dafür gegeben, daß die einander formentsprechenden Gleitflächen der beiden Komponenten genau passend zusammenwirken.

Fig. 2 zeigt diejenige Ausführungsvariante, bei welcher die einstückige Femurkomponente 3 durch ein Paar von Kondylenschalen 10 ersetzt ist, die genau gleich den Kondylenteilen 5 der einstückigen Femurkomponente 3 geformt sind; sie sind sozusagen von der einstückigen Komponente abgeschnitten. Ihre Gleitflächen 6 entsprechen genau den Gleitflächen 6 der einstückigen Komponente und wirken in der gleichen Weise mit den Gleitmulden 7 des Tibiaplateaus zusammen, vorausgesetzt, daß ihre relative Positionierung genau derjenigen der Kondylenteile 5 der einstückigen Komponente entspricht. Die Prothesenteile sind in mehreren Größenstufen verfügbar.

Die dem Knochen zugewendeten Verankerungsflächen der Femurkomponenten 3 und 10 sind übereinstimmend aus Ebenen 11, 12, 13 polygonal zusammengesetzt, die übereinstimmenden Resektionensflächen am Knochen entsprechen. Es sind auch an übereinstimmender Stelle befindliche Verankerungszapfen 14 vorgesehen. Die dem Patellaschild 4 zugeordneten Resektions- und Verankerungsflächen der Komponente 3 entfallen naturgemäß bei den Kondylenschalen 10.

Zum Resezieren der Femurkondylen dient die in Fig. 3 dargestellte Sägelehre 20, die als solche bekannt ist (WO 98/53747) und daher keiner detaillierten Beschreibung bedarf. Sie enthält zwischen Seitenwangen 21 mehrere Paare von Führungsstangen 22 zur Festlegung der Ebenen, in denen eine Knochensäge zur Herstellung der Resektionsschnitte geführt wird. Wenn die einstückige Komponente 3 verwendet werden soll, erstrecken sich die Schnitte über die ganze Breite des knöchernen Kondylenpaars. Wenn lediglich Kondylenschalen 10 angesetzt werden sollen, wird die Sägelehre 20 mit einem Sperreinsatz 23 versehen, der den Zwischenkondylenbereich schützt. Seine Seitenflächen sind parallel und fluchten mit denjenigen Linien, denen die inneren Seitenränder der Kondylenschalen in der vorgesehenen Position folgen sollen. Der Sperreinsatz 23 schützt den Zwischenkondylenbereich vor unerwünschten Schnitten. Es sind mehrere Sperreinsätze verfügbar, jeweils passend zu einer Größenstufe der Prothese.

Zum positionsgerechten Ansetzen der Kondylenschalen 10 an den Femurknochen dient das in Fig. 4 dargestellte Einsetzinstrument 30. Es besteht aus einem Griff 31, einer Halterungsplatte 32 und zwei Klemmen 33 zur Aufnahme der Kondylenschalen 10. Um die Teile bequemer zusammensetzen zu können, ist ein Sockel 34 vorgesehen, der eine vertikale Bohrung passend zu dem Durchmesser des Griffteils 31 enthält und der das Instrument während des Aufsetzens der Kondylenschalen und der Klemmen in der dargestellten Lage hält.

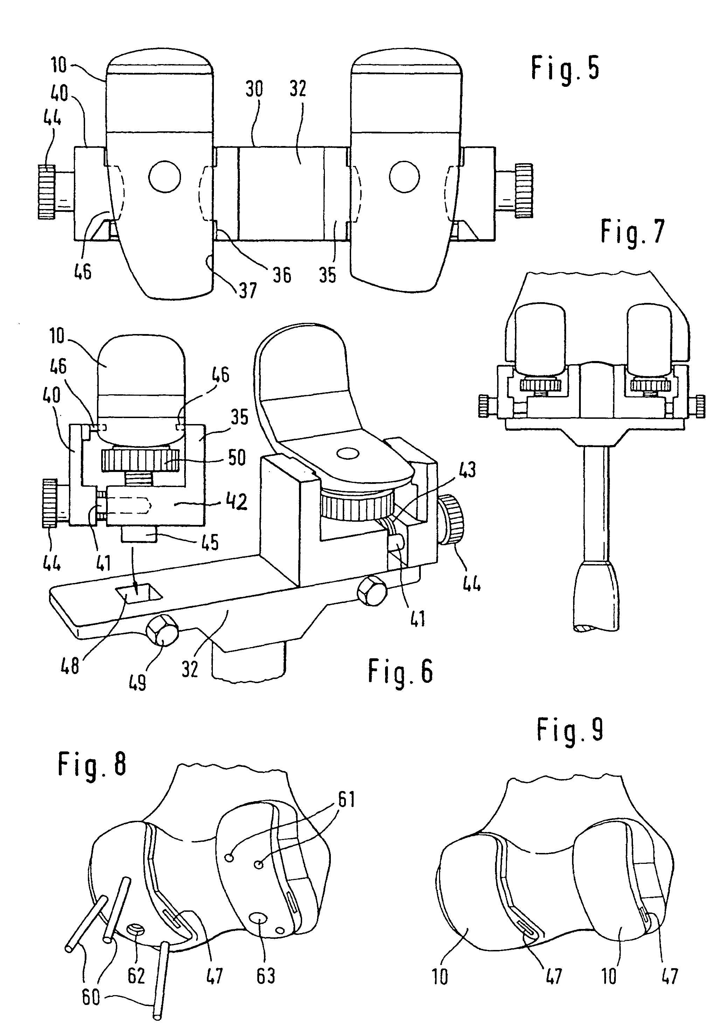

Wie man Fig. 5 entnimmt, weisen die Klemmen 33 innenseitige Klemmbacken 35 auf, die parallele Anschlußflächen 36 für die Seitenflächen 37 der Kondylenschalen bilden. Das Zusammenwirken der Flächen 36 mit den Seitenflächen 37 sorgt für eine genau parallele Ausrichtung der Schalen 10. Es versteht sich, daß dieses Ziel auch dann erreichbar wäre, wenn diese Fläche nicht geradlinig gestaltet wären; jedoch erleichtert die geradlinige Form die Herstellung sowie die optische Kontrolle des richtigen Schalensitzes. Zudem wird sie den anatomischen Verhältnissen gerecht.

Auf ihren Außenseiten werden die Klemmen 33 von einer beweglichen Backe 40 gebildet, die parallel zu der feststehenden, inneren Backen 35 geführt ist. Die Führung geschieht im dargestellten Beispiel durch Führungsstangen 41, die mit entsprechenden Bohrungen des Klemmensockels 42 zusammenwirken, der starr mit der inneren, feststehenden Backe 35 verbunden ist. Die Vorstellung geschieht mittels einer Gewindespindel 43 und eines außen damit verbundenen Drehknopfs 44. Die Bakken 35, 40 sind mit einander zugewendeten Vorsprüngen 46 versehen. Die Ränder der Kondylenschalen enthalten Ausnehmungen 47, die zu diesen Vorsprüngen passen. Die Vorsprünge und Ausnehmungen sind länglich ausgeführt und zwingen dadurch den Kondylenschalen im Einsetzinstrument eine vorbestimmte Richtung auf.

Von der Unterseite des Klemmensockels 42 ragt ein Zapfen 45 vor, der passend zu je einer Aufnahmeöffnung 48 in der Halterungsplatte 32 ausgebildet ist. Der Zapfen 45 und die Aufnahmeöffnung 48 sind im Verhältnis zueinander undrehbar ausgebildet, im dargestellten Beispiel dank einem quadratischen Querschnitt. Die Zapfen können mittels Schrauben 49 in den Aufnahmeöffnungen gesichert werden. Die beiden Aufnahmeöffnungen 48 in der Halterungsplatte 32 verlaufen parallel zueinander.

Für jede zum Prothesensystem gehörige Größenstufe ist eine gesonderte Halterungsplatte 32 vorgesehen. Die Halterungsplatte unterschiedlicher Größenstufen unterscheiden sich durch ihre Breite in LM-Richtung und durch den Abstand ihrer Aufnahmeöffnungen 48. Die zugehörigen Klemmen 33 können übereinstimmen. Statt dessen wäre es auch möglich, unterschiedliche Klemmen zu verwenden und ein und dieselbe Halterungsplatte, wobei die Anordnung der Zapfen 47 an den Klemmen für den gewünschten Abstand derselben voneinander sorgen würde. Schließlich ist es auch denkbar, lediglich eine Halterungsplatte zu verwenden, die eine größere Anzahl von Aufnahmeöffnungen 48 aufweist, die es gestatten, Klemmen mit unterschiedlichem Abstand aufzusetzen.

Jede Klemme 33 enthält zwischen den Backen 35, 40 den breit ausgebildeten Kopf 50 einer im Klemmensockel 42 geführten Stellschraube. Die Oberfläche des Kopfs 50 ist mit einem nachgiebigen Material belegt, beispielsweise Polyethylen. Nachdem eine Kondylenschale 10 zwischen den Backen 35, 40 geklemmt worden ist, wird die Stellschraube 50 gegen die ihr zugewendete Gleitfläche geschraubt und stützt diese. Sie wird in den Ansprüchen deshalb auch als Gleitflächenstütze bezeichnet. Sie dient dazu, beim Aufsetzen der Schalen auf den Knochen eine Kraftübertragung von dem Einsetzinstrument auf die Kondylenschalen in Aufsetzrichtung zu ermöglichen, ohne daß die Kräfte von den Vorsprüngen 46 übertragen werden müssen. Außerdem tragen die Gleitflächenstützen 50 zur korrekten Positionierung der Kondylenschalen im Einsetzinstrument bei, falls die bewegliche Klemmbacke 40 Spiel gegenüber der anderen Klemmbacke 35 aufweist oder wenn die Vorsprünge 46 Spiel gegenüber den sie aufnehmenden Vertiefungen 47 haben.

Wenn ein Paar von Kondylenschalen 10 auf den resezierten Knochen aufgesetzt werden soll, werden sie zunächst in der erläuterten Weise von dem Einsetzinstrument gefaßt. Dieses wird dann, wie in Fig. 7 gezeigt, mit den Kondylenschalen auf den Knochen aufgesetzt. Sobald die Kondylenschalen fixiert sind, wird zunächst die Halterungsplatte 32 mit dem Griffteil 31 des Einsetzinstruments abgenommen, indem die Schrauben 49 gelöst werden und die Halterungsplatte 32 von den Klemmen 33 und deren Zapfen 45 abgezogen wird. Dies ist deshalb möglich, weil beide Klemmen 33 mit der Halteplatte 32 in derselben Löserichtung ihrer Zapfen 45 verbunden sind. Anschließend können die Klemmen 33 von den Schalen gelöst werden, indem die bewegliche Backe 40 zurückgeschraubt wird, nachdem gegebenenfalls zuvor die Gleitflächenstütze 50 gelockert wurde.

Wenn es sich bei den Kondylenschalen um die endgültigen Prothesenteile handelt, wird das Einsetzwerkzeug abgenommen, nachdem sie hinreichend mit Zement oder zementfrei am Knochen verankert wurden. Wenn es sich um Probeimplantate handelt, werden sie zuvor am Knochen mittels Fixierstiften 60 befestigt, für die in den Kondylenschalen Bohrungen 61 vorgesehen sind. Diese liegen außerhalb des von den Klemmen abgedeckten Bereichs, so daß die Stifte 60 eingesetzt werden können, solange die Prothesenschalen noch durch das Einsetzinstrument fixiert sind. An derjenigen Stelle, wo die Implantate einen Verankerungszapfen 14 aufweisen, enthalten die Probeimplantate eine Bohrung 62 entsprechender Größe. Wenn nicht schon vorher im Knochen eine Bohrung zur Aufnahme der Zapfen eingebracht worden war, kann diese durch die Bohrung 62 der Probeimplantate an der richtigen Stelle vorgenommen werden. Danach wird in die Bohrung 62 und die darunter befindliche Knochenbohrung ein Fixierbolzen 63 eingedrückt, der nun die Probeimplantate anstelle der entfernten Fixierstifte 60 zu halten vermag und dessen Kopf in der Bohrung 62 verschwindet. Die Implantatsstellung und die Gelenkfunktion kann nun mit Hilfe der Probeimplantate überprüft werden. Nachdem diese entfernt wurde, werden die endgültig zu verwendenden Prothesenschalen in der gleichen Weise angesetzt.

## Patentansprüche

1. Knieprothesensystem, umfassend ein einstückiges Tibiaplateau (2) mit zwei Gleitmulden (7) und eine einstückige Femurkomponente (3) mit zwei Kondylengleitflächen (6), deren Abstand und Form auf die tibialen Gleitmulden (7) abgestimmt sind, **dadurch gekennzeichnet, daß** das System als Alternative zu der einstückigen Femurkomponente (3) ein Paar von unverbundenen, femoralen Kondylenschalen (10), deren Gleitflächenform auf die tibialen Gleitmulden (7) abgestimmt ist, und ein Einsetzinstrument (30) umfaßt, das den Kondylenschalen (10) eine auf die tibialen Gleitmulden (7) abgestimmte relative Position verleiht, in welcher die Lage der kondylären Verankerungsflächen (11, 12, 13) der Kondylenschalen (10) mit derjenigen der einstückigen Femurkomponente (3) übereinstimmt.

2. Knieprothesensystem nach Anspruch 1, **dadurch gekennzeichnet, daß** die Form und der Abstand der Gleitflächen (6) der Kondylenschalen (10) mit der Form und dem Abstand der Kondylengleitflächen (6) der einstückigen Femurkomponente (3) übereinstimmen.

3. Knieprothesensystem nach Anspruch 1 oder 2, **dadurch gekennzeichnet, daß** es eine Sägelehre (20) umfaßt, deren Schnittebenen sowohl zu der einstückigen Femurkomponente (3) als auch zu den Kondylenschalen (10) passen.

4. Knieprothesensystem nach Anspruch 3, **dadurch gekennzeichnet, daß** die Sägelehre (20) einen entfernbaren Sperreinsatz (23) im Zwischenkondylenbereich (24) aufweist.

5. Knieprothesensystem nach einem der Ansprüche 1 bis 4, **dadurch gekennzeichnet, daß** das Einsetzinstrument (30) zu den im Einbauzustand einander zugekehrten Seitenflächen (37) der Kondylenschalen (10) passende, parallele Anschlußflächen (36) aufweist.

6. Knieprothesensystem nach Anspruch 5, **dadurch gekennzeichnet, daß** der gegenseitige Abstand der parallelen Anschlußflächen (36) des Einsetzinstruments (30) stufenweise entsprechend den unterschiedlichen Abständen, die den Kondylenschalen (10) in unterschiedlichen Größenstufen des Prothesensystems zugeordnet sind, verstellbar ist.

7. Knieprothesensystem nach Anspruch 6, **dadurch gekennzeichnet, daß** die Verstellbarkeit auf der Verwendung von Wechselteilen (32) beruht.

8. Knieprothesensystem nach einem der Ansprüche 5 bis 7, **dadurch gekennzeichnet, daß** die Anschlußflächen (36) von je einer feststehenden Backe (35) von separaten, den Kondylenschalen (10) jeweils zugeordneten Klemmen (33) gebildet sind.

9. Knieprothesensystem nach Anspruch 8, **dadurch gekennzeichnet, daß** zur Aufnahme der Klemmen (33) unterschiedlich breite Halterungen (32) vorgesehen sind.

10. Knieprothesensystem nach Anspruch 9, **dadurch gekennzeichnet, daß** beide Klemmen (33) in derselben Löserichtung von der Halterung (32) abnehmbar sind.

11. Knieprothesensystem nach einem der Ansprüche 8 bis 10, **dadurch gekennzeichnet, daß** die Kondylenschalen (10) und die Klemmen (33) mit zusammenwirkenden, sie gegenseitig positionierenden Erhöhungen (46) und Vertiefungen (47) versehen sind.

12. Knieprothesensystem nach einem der Ansprüche 8 bis 11, **dadurch gekennzeichnet, daß** die Backen (35, 40) der Klemmen (33) in LM-Richtung auf die Kondylenschalen (10) einwirken und mit einer verstellbaren Gleitflächenstütze (50) versehen sind.

13. Knieprothesensystem nach einem der Ansprüche 1 bis 12, **dadurch gekennzeichnet, daß** Probe-Kondylenschalen vorgesehen sind, die außerhalb ihres von dem Einsetzinstrument (30) erfaßten Bereichs Bohrungen (61) für Fixierstifte (60) aufweisen.

14. Knieprothesensystem nach Anspruch 13, **dadurch gekennzeichnet, daß** die Kondylenschalen (10) wenigstens einen Verankerungszapfen (14) aufweisen und die Probe-Kondylenschalen an der Stelle dieses Zapfens eine Bohrung (62) für einen Fixierbolzen (63) mit in der Bohrung (62) versenkbarem Kopf enthalten.

## Claims

1. A knee prosthesis system, comprising a one-piece tibial plateau (2) with two sliding depressions (7) and a one-piece femur component (3) with two condyle slide surfaces (6) whose distance apart and shape are adapted to the tibial sliding depressions (7), **characterised in that** the system comprises as an alternative to the one-piece femur component (3) a pair of unconnected femoral condyle shells (10), the slide surface shape of which is adapted to the tibial sliding depressions (7), and comprises an insertion instrument (30) which imparts to the condyle shells (10) a relative position adapted to the tibial sliding depressions (7), in which the position of the condylar anchoring surfaces (11,12,13) of the condyle shells (10) coincides with that of the one-piece femur component (3).

2. A knee prosthesis system according to Claim 1, **characterised in that** the shape and the distance apart of the slide surfaces (6) of the condyle shells (10) coincide with the shape and distance apart of the condyle slide surfaces (6) of the one-piece femur component (3).

3. A knee prosthesis system according to Claim 1 or 2, **characterised in that** it comprises a saw jig (20) whose cutting planes match both the one-piece femur component (3) and the condyle shells (10).

4. A knee prosthesis system according to Claim 3, **characterised in that** the saw jig (20) has a removable locking insert (23) in the intercondyle region (24).

5. A knee prosthesis system according to any one of Claims 1 to 4, **characterised in that** the insertion instrument (30) has parallel abutting surfaces (36) matching the lateral surfaces (37) of the condyle shells (10) facing one another in the fitting condition.

6. A knee prosthesis system according to Claim 5, **characterised in that** the mutual distance apart of the parallel abutting surfaces (36) of the insertion instrument (30) is adjustable stepwise corresponding to the different distances which are allocated to the condyle shells (10) in different size steps of the prosthesis system.

7. A knee prosthesis system according to Claim 6, **characterised in that** the adjustability is based on the use of interchangeable parts.

8. A knee prosthesis system according to any one of Claims 5 to 7, **characterised in that** the abutting surfaces (36) of a respective stationary jaw (35) are formed by separate clamps (33) respectively associated with the condyle shells (10).

9. A knee prosthesis system according to Claim 8, **characterised in that** mountings (32) of different width are provided to accommodate the clamps (33).

10. A knee prosthesis system according to Claim 9, **characterised in that** both clamps (33) can be removed from the mounting (32) in the same release direction.

11. A knee prosthesis system according to any one of Claims 8 to 10, **characterised in that** the condyle shells (10) and the clamps (33) are provided with co-operating elevations (46) and recesses (47) which mutually locate them.

12. A knee prosthesis system according to any one of Claims 8 to 11, **characterised in that** the jaws (35,40) of the clamps (33) act on the condyle shells (10) in the LM direction and are provided with adjustable slide surface supports (50).

13. A knee prosthesis system according to any one of Claims 1 to 12, **characterised in that** test condyle shells are provided which have bores (61) for locating pins (60) outside their region defined by the insertion instrument (30).

14. A knee prosthesis system according to Claim 13, **characterised in that** the condyle shells (10) have at least one anchoring peg (14) and the test condyle shells comprise at the location of these pegs a bore (62) for a locating pin (63) with a head which can be lowered in the bore (62).

## Revendications

1. Système pour prothèse de genou, comprenant un plateau tibial monobloc (2), muni de deux cavités de glissement (7), et une composante fémorale monobloc (3), munie de deux surfaces de glissement condyliennes (6), dont la distance et la forme sont adaptées à celles des cavités de glissement tibiales (7), **caractérisé en ce que**, en variante de la composante fémorale monobloc (3), le système comporte une paire de coquilles condyliennes fémorales (10) non reliées, dont la forme des surfaces de glissement est adaptée à celle des cavités de glissement tibiales (7), et un instrument inséré (30), par lequel les coquilles condyliennes (10) sont amenées dans une position relative ajustée à celle des cavités de glissement tibiales (7), dans laquelle la position des surfaces d'ancrage condyliennes (11, 12, 13) des coquilles condyliennes (10) correspond à celle de la composante fémorale monobloc (3).

2. Système pour prothèse de genou selon la revendication 1, **caractérisé en ce que** la forme et la distance des surfaces de glissement (6) des coquilles condyliennes (10) correspondent à la forme et à la distance des surfaces de glissement condyliennes (6) de la composante fémorale monobloc (3).

3. Système pour prothèse de genou selon la revendication 1 ou 2, **caractérisé en ce qu'**il comporte un gabarit de coupe (20) dont le plan de coupe est adapté non seulement à la composante fémorale monobloc (3), mais aussi aux coquilles condyliennes (10).

4. Système pour prothèse de genou selon la revendication 3, **caractérisé en ce que** le gabarit de coupe (20) comporte un insert de blocage (23) amovible dans la zone intercondylienne (24).

5. Système pour prothèse de genou selon l'une quelconque des revendications 1 à 4, **caractérisé en ce que** l'instrument inséré (30) comporte des surfaces de jonction parallèles, adaptées aux surfaces latérales (37) des coquilles condyliennes (10), orientées l'une vers l'autre à l'état monté.

6. Système pour prothèse de genou selon la revendication 5, **caractérisé en ce que** la distance réciproque entre les surfaces de jonction (36) parallèles de l'instrument inséré (30) est réglable graduellement en fonction des différentes distances entre les coquilles condyliennes (10) associées aux différents niveaux de taille du système de prothèse.

7. Système pour prothèse de genou selon la revendication 6, **caractérisé en ce que** la possibilité de réglage se fonde sur l'utilisation de pièces interchangeables (32).

8. Système pour prothèse de genou selon l'une quelconque des revendications 5 à 7, **caractérisé en ce que** les surfaces de jonction (36) sont formées chacune par une joue (35) fixe de mâchoires de serrage (33) séparées, associées chacune à une coquille condylienne (10).

9. Système pour prothèse de genou selon la revendication 8, **caractérisé en ce que** des supports (32) de différentes largeurs sont prévus pour la réception des mâchoires de serrage (33).

10. Système pour prothèse de genou selon la revendication 9, **caractérisé en ce que** les deux mâchoires de serrage (33) peuvent être retirées du support (32) dans la même direction de retrait.

11. Système pour prothèse de genou selon l'une quelconque des revendications 8 à 10, **caractérisé en ce que** les coquilles condyliennes (10) et les mâchoires de serrage (33) sont munies de saillies (46) et de creux (47) coopérants et positionnés face à face.

12. Système pour prothèse de genou selon l'une quelconque des revendications 8 à 11, **caractérisé en ce que** les joues (35, 40) des mâchoires de serrage (33) agissent sur les coquilles condyliennes (10) dans la direction LM et sont munies d'un appui pour surface de glissement (50) réglable.

13. Système pour prothèse de genou selon l'une quelconque des revendications 1 à 12, **caractérisé en ce que** des coquilles condyliennes d'essai sont prévues, lesquelles comportent, en dehors de leur zone saisie par l'instrument inséré (30), des perçages (61) pour des chevilles de blocage (60).

14. Système pour prothèse de genou selon la revendication 13, **caractérisé en ce que** les coquilles condyliennes (10) comportent au moins un plot d'ancrage (14) et les coquilles condyliennes d'essai contiennent à l'emplacement de ce plot d'ancrage un perçage (62) pour un boulon de blocage (63) dont la tête pénètre dans le perçage (62).
